Europäisches Patentamt

European Patent Office

Office européen des brevets

(11) Publication number: 0 396 108
A2

# EUROPEAN PATENT APPLICATION

(21) Application number: 90108279.2

(51) Int. Cl.5: A61F 13/15

(22) Date of filing: 30.04.90

(30) Priority: 02.05.89 BR 8902476

(43) Date of publication of application:
07.11.90 Bulletin 90/45

(84) Designated Contracting States:
AT BE CH DE ES FR GB IT LI LU NL

(71) Applicant: McNEIL-PPC INC.
Van Liew Avenue
Milltown New Jersey 08850(US)

(72) Inventor: Gottschalk,Carlos Alberto
Av. Engenheiro Edgar Egidio de Souza 373
apto. 202
Sao Paulo,SP(BR)

(74) Representative: Strehl, Schübel-Hopf,
Groening
Maximilianstrasse 54 Postfach 22 14 55
D-8000 München 22(DE)

(54) Absorbent structure and absorbent product.

(57) Absorbent structures for disposable products, preferably for sanitary pads, comprise a composite nonwoven covering overlapping an absorbent panel (1), the outer layer (3) of said covering, i.e., the one that is in contact with the skin, being of a hydrophobic material and perforated, and the inner layer (2) of said covering, which is located between the absorbent panel (1) and the outer layer (3), being limitedly hydrophilic and essentially without perforation. The layers that make up the covering are not interbonded along the body fluid discharge surface.

FIG 1

EP 0 396 108 A2

## ABSORBENT STRUCTURE AND ABSORBENT PRODUCT

The present invention refers to a structure for absorbing body fluids, in disposable articles such as diapers, sanitary pads, bandages, etc.

More particularly, it refers to sanitary pads without prejudice to other applications that can profit from the teaching disclosed herein.

Thus, in the following text, reference is made to sanitary pads to facilitate understanding, without excluding other embodiments. Sanitary pads, also known as intimate pads or feminine hygienic pads, are articles known and widely used in modern society. They are intended for absorbing menstrual fluids or other vaginal discharges outside the menstrual period. They are called disposable because they are not used a second time.

The basic structure of these products is made up of three layers, namely:
- the layer that is in contact with the user's body, or covering, which is intended for rapidly transferring the fluid to the absorbent layer and is usually made of a nonwoven polymer fiber base fabric;
- the absorbent layer, or absorbent panel, made up of a material having a strong affinity for liquids, the function of which is to absorb and hold the fluid that goes through the covering. It is traditionally made of cellulose pulp;
- the layer that comes after the absorbent panel, or lining; it is traditionally made of an impermeable thermoplastic polymeric film, for instance polyethylene. It is intended for preventing the liquid held in the absorbent panel from leaking.

Each of these layers has been the object of developments to improve the product with a view to providing the user with more comfort, from the point of view of both the implicit performance of the materials - for instance, a greater absorption capacity - and more subjective aspects - for instance, a greater sensation of safety against leakage during use.

The present invention provides an improvement of the performance by the development of a covering designed for sanitary pads, by reducing leakage and improving more subjective properties such as the absence of the feeling of moisture during use and a greater feeling of cleanliness by masking the coloration of the absorbent panel impregnated with menstrual fluid.

In the prior art, many attempts have been made to improve the performance of sanitary pads by the use of composite structures.

Commercial products are known in which the outer layer is made of a nonwoven hydrophilic or hydrophobic fabric, and the inner layer is made of a material having a great capacity of distributing fluid, for instance a panel, or a material that is more hydrophilic than the absorbent panel.

Such a structure is subject to frequent leakage, since one or more of the following situations occurs:
- the fluid rapidly spreads over the whole inner layer, due to the great capacity of spreading, and quickly reaches the edges;
- the fluid is held quite intensely in the inner layer, since its affinity for the latter is greater than for the absorbent panel;
- the inner layer rapidly saturates and any additional fluid discharged onto it will overflow toward the sides.

The fact that the absorbed fluid remains close to the outer surface of the structure, thus giving a vivid appearance of the blood held therein, which may not be pleasing to the user, and still maintaining the feeling of moisture during the use, is another problem.

Other cases known from the prior art are those in which the outer layer of the covering, that is to say, the one that is in contact with the user's body is either totally or partially hydrophilic. The following patents fall into this category: U.S. 3,595,235 of Georgia Pacific Corp., U.S. 4,519,799 of Kao Corp., U.S. 4,240,416 of Benecke GMBH, U.S. 4,704,112 of Unicharm Corp., U.S. 3,888,254 of Hendricks L., U.S. 4,324,247 of Procter & Gamble and U.S. 4,417,893 of Kao Corp. Many of these patents also disclose gluing between the two layers of the covering, but this is not the case of the present invention.

Independently of the properties of the innermost layer of the covering according to these patents, since the surface that is in contact with the user's body is either totally or partially hydrophilic, the sensation of moisture and the coloration of the blood in the product are points that causes displeasure and can lead to the impression that the product does not fulfill its implicit function, namely, that of absorbing menstrual fluids.

Thus, as is the case of the present invention, one finds in the prior art composite coverings for sanitary pads whose outer layer, the one that is in contact with the user's body, is hydrophobic. For instance, Patent PI 75,248 of Gesfor AG differs from this invention by creating a structure in which the covering is made up by two permeable hydrophobic films. In the same way, U.S. Patent 4,636,209 of Kimberly Clark differs from the present invention, since the inner layer, i.e., the one that is between the absorbent panel and the layer in contact with the user's body, has some fibers oriented vertically with respect to the horizontal plane, that is to say, it is a sophisticated and expensive material, unnecessary according to this invention.

The teachings of Patent PI 8102867 of Procter & Gamble also differ from the present invention since, although the outer layer of the covering is a perforated, hydrophobic plastic film, the next fiber layer is attached to the inner layer, thus requiring a later unnecessary operation.

One of the advantages of the invention over the prior art is the face that a surprising performance is obtained without the need for resorting either to sophisticated materials or to adhering the layers of the covering to each other.

Thus, the invention relates to an absorbent structure characterized by containing a nonwoven composite covering on an absorbent panel, the outer layer of said covering being substantially hydrophobic and perforated, and the inner layer of said covering being limitedly hydrophilic and essentially non-perforated.

The outer layer of the covering is the one that is in contact with the user's body. The inner layer of the covering is the one that is inserted between the absorbent panel and the outer layer.

As used herein, the expression "essentially hydrophobic" refers to materials that have a relative absorbency, as determined by the Sinking Basket method, of more than 2 hours, preferably more than 4 hours.

Next, a description of the method of determining the relative absorbency is given (also known as Sinking Basket): place 5g of a sample in a wire basket (the diameter of the wire being 0.8mm, the diameter of the basket being 5cm, its height being 8cm, having divisions every 2c, a weight of 3.0 + or - 0.1g). Put the basket with the sample in a horizontal position 5cm above the distilled water contained in a beaker of 2000ml at 25 + or - 1°C. Release the basket and set off the chronometer at the moment when the basket submerges completely. The reading of the time is the measure of the relative absorbency.

Any mention of the relative absorbency in the following text relates to the "sinking basket" test.

The outer nonwoven layer of the covering according to the invention is perforated by any known means, for instance, by using jets of water or air, by mechanical methods such as cold or hot perforation by pins, etc. Since the hydrophobic layer is perforated, it becomes fluid permeable. Preferably, the perforation density should be in the range of from 88 to 225 holes/square inch, which is the most adequate value for use with menstrual fluid.

By "limitedly hydrophilic" is meant that the material of the inner layer of the covering of the invention has a time of relative absorbency of from 1.5 to 10 seconds, preferably from 1.5 to 5 seconds, advantageously between 1.5 and 2.5 seconds. The inner layer of the covering of the invention is preferably thermobonded by known methods, for instance by the meltblown or spunbonding methods, without excluding other types of sealing - for instance with a binder bath given to carded fibers, followed by calendering and drying (known as "card and bind").

As those skilled in the art know, the hydrophilic nature inherent in the product can be altered, depending upon its density. For example, by treatment with surfactants.

Other characteristics of the nonwoven fabrics used in the two layers of the covering of the invention, such as softness, the basic weight, resistance, etc., are easily determined by those skilled in the art in function of the desired utilization.

By "essentially non-perforated" is meant that the inner layer of the covering according to the invention is preferably continuous, without perforation. However, it can have small perforations having, for example, 1/10 of the diameter of the holes of the outer layer.

The two layers that form the absorbent covering of the invention are not bonded or interconnected, in any way along the fluid-discharge surface, whereby one step and an additional cost in the obtention of the product are eliminated. Such layers could be joined but the gain in performance does not justify the procedure.

The absorbent panel of the invention is preferably made of bleached wood-fiber pulp or any other absorbent material known from the prior art that has a relative absorbency time of from 0 to 1.5 seconds.

Although without theoretical backing, the Applicant believes that it has reached an ideal balance between the properties and parameters of the components with its invention, that is to say: an increasing hydrophilicity gradient of the outer layer towards the absorbent panel, in combination with the permeability of the layers of the covering, so that body fluids, and in particular menstrual fluid - while respecting its characteristic HLB (hydrophilic-lipophilic balance) values - will be rapidly led through the covering and readily absorbent by the absorbent panel.

The return of fluid absorbed in the absorbent panel to the surface of the covering is greatly inhibited by the inner layer of the covering, which is designed for providing a rapid penetration, a low return of wetness (wetback) and reduced stain area after wetback.

The performance obtained by the cumulative action of the layers of the covering then provides absorption efficiency and conveys a feeling of security to the user, be it by the absence of any sensation of wetness (the outer layer is hydrophobic and retains virtually no wetness) or by the aspect of cleanliness (the absorbed fluid stays away from the discharge surface and is covered by two layers of nonwoven fabric).

The invention further refers to an absorbent article, preferably a sanitary pad, characterized by being made·up of the following elements:
- a composed nonwoven fabric covering, the outer layer being essentially hydrophobic and perforated, and the inner layer being limitedly hydrophilic and essentially non-perforated;
- an absorbent panel;
- a lining of a flexible and liquid impermeable material.

Preferably, the layers are not attached or interconnected in any way along the surface that receives the discharge of fluid.

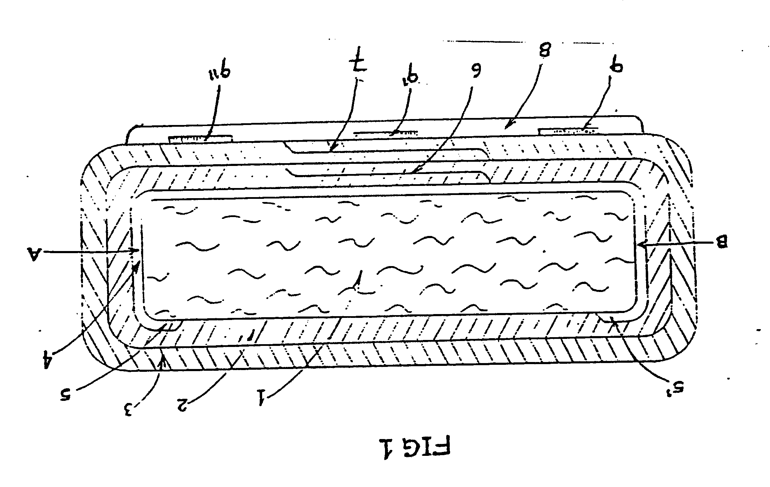

A particular embodiment of the invention is illustrated in Figure 1 attached hereto.

The figure comprises a cross section of an absorbent pad having the dimensions of its components enlarged and/or distorted with a view to facilitate understanding.

The rear face of a wood-fiber pulp panel 1 as well as its sides A and B which represent its thickness and, furthermore, a minor portion of the width of the panel, as shown at 5 and 5', is covered by a lining 4 of thermoplastic film, for example, of polyethylene.

The covering is made up of the inner layer 2 and outer layer 3 that completely surrounds the absorbent panel 1 and the lining 4. The layers 2 and 3 are overlapped and sealed at the back portion of the product, as can be seen at 6 and 7.

The outer layer 3 is longer than the absorbent panel 1 and the inner layer 2, it being sealed onto itself at the ends (not shown in the cross section of Figure 1) of the product (the ends being those where the length of the product begins and ends).

The bonds mentioned above are of any known kind, for instance, by applying a liquid adhesive, hot melt or internal sealing.

In the particular embodiment, the product receives three strips of pressure sensitive adhesive 9,9' and 9" applied along the length of the layer 3 on the back face of the product, covered by a strip 8 of a removable protecting material, for instance siliconated paper, which is removed by the user for adhering the product inside her underclothes for use.

A practical example of utilization of the described invention will now be given, merely for the purpose of illustration and without the intention of imposing any limitation.

378 sanitary pads were tested in-vivo with a group of 55 users, 183 of them being in accordance with the invention and 195 as available on the market, taken as a standard. The test of the product was carried out in accordance with the habit of each user during her menstruation period, alternating the product of the invention and the standard one.

The product according to the invention corresponds to what is described in Figure 1 with the following characteristics:
- covering/outer layer: a polyester nonwoven web with textile treatment to obtain a relative absorbency time of more than 4 hours, a basic weight of 22 g/square meter and 144 holes/square inch using the card-and-bind obtention method.
- inner layer: a polypropylene nonwoven web with a textile treatment of the fibers that make it up, to obtain a relative absorbency time of 2 seconds, a basic weight of 20 g/square meter, thermobonded by calendering with a pattern of 36 points/square centimeter at 25% of molten area;
- absorbent panel: 100% wood fiber fluff pulp, approximate weight of 9g, approximate dimensions 1.6 x 6.0 x 20cm.;
- lining: polyethylene film, 0.02mm thick and with a basic weight of 19 g/square meter.

Hot sealing was used (thermobondings).

The standard product is equivalent, with the following differences as compared with the covering described above:
- outer layer: the nonwoven web being 100% rayon, with a basic weight of 19 g/square meter, a relative absorbency time close to 0, cluster perforation of 15 bores of about 1mm in diameter inscribed in circumferences of about 10 mm in diameter, the imaginary 30 mm-side square pattern being repeated indefinitely, the squares containing a circumference at each vertex and one in the center;
- inner layer: a bleached cellulose additiveless fiber paper containing 20% of short fibers and 80% long fibers, with a basic weight of 20 g/square meter, a porosity of 4 seconds/100 ml (32 layers/300 ml of air). The results obtained are the following:

1 - a reduction in the leakage rate of 19.5% of the standard to 9.8% of the product of the present invention (95% statistical reliability);

2 - an indication that there was a lesser feeling of wetness during the use of the product of the invention;

3 - an indication that the product of the invention appeared cleaner after use.

From these results, it follows that the absorbent structure of the invention, which is simple and innovatory, provides a greater efficiency, a lesser feeling of wetness and a greater masking of the absorbed fluid.

## Claims

1. An absorbent structure characterized by containing a composite nonwoven fabric covering overlapping an absorbent material (1), the outer layer (3) of said covering being essentially hydrophobic and perforated, and the inner layer (2) being hydrophilic and essentially non-perforated.

2. An absorbent structure according to Claim 1, characterized in that: the relative absorbency time of said outer layer (3) is greater that 2 hours; the relative absorbency time of said inner layer (2) being in the range of from 1.5 to 10 seconds; the relative absorbency time of said absorbent material (1) is in the range of from 0 to 1.5 seconds; and said outer layer (3) has from 35 to 300 holes/square inch.

3. An absorbent structure according to Claim 2, characterized in that said inner layer (2) is obtained preferably by thermobonding.

4. An absorbent structure according to Claim 2, characterized in that said outer and inner layers (3,2) are not interbonded along the fluid-discharge surface.

5. An absorbent structure according to Claim 2, characterized in that said inner layer (2) is continuous and without perforation.

6. An absorbent structure according to Claim 2, characterized in that the relative absorbency time of said outer layer (3) is greater than 4 hours.

7. An absorbent structure according to Claim 2, characterized in that the relative absorbency time of said inner layer (2) is preferably in the range of from 1.5 to 5 seconds.

8. An absorbent structure according to Claim 2, characterized in that the relative absorbency time of said inner layer (2) is preferably in the range of from 1.5 to 2.5 seconds.

9. An absorbent structure according to Claim 2, characterized in that said outer layer (3) has preferably from 50 to 250 holes/square inch.

10. An absorbent structure according to Claim 2, characterized in that said outer layer (3) has preferably from 88 to 225 holes/square inch.

11. A disposable absorbent product characterized by being made up of the following elements:
- a composite nonwoven covering, the outer layer (3) of said covering being essentially hydrophobic and the inner layer (2) of said covering being limitedly hydrophilic and essentially non-perforated;
- an absorbent panel (1); - a lining (4) made of flexible and fluid impermeable material.

12. An absorbent product according to Claim 11, characterized in that said absorbent product is a menstrual fluid absorbing product.

13. An absorbent product according to Claim 12, characterized in that a lining (4) covers the rear face of said pulp panel (1), the sides (A,B) and a portion of the width (5,5$'$) of said panel (1); said covering is made up by overlapping inner (2) and outer (3) layers, which are bonded at the back side (6,7) of the product; the outer layer (3) is bonded onto itself at the ends of the length of said product; the outer layer (3) has, at the back part of the product, three longitudinal strips of a pressure sensitive adhesive (9.9$'$, 9$''$) covered by a removable strip of protecting material (8).

FIG1